# EUROPEAN PATENT APPLICATION

(11) **EP 2 594 309 A1**
(43) Date of publication of application: **22.05.2013**
(21) Application number: 12190656.4
(22) Date of filing: 30.10.2012
(51) Int. Cl.: A61M 25/06

(54) **Needle for medical applications**

(30) Priority: 18.11.2011 DE 202011052035 U
(71) Applicant: Schwarzbich, Jörg, 33615 Bielefeld (DE)
(72) Inventor: Schwarzbich, Jörg, 33615 Bielefeld (DE)
(74) Representative: Ter Meer Steinmeister & Partner

(57) **Abstract**

A needle for piercing the skin of a patient, comprising a protective sleeve (20) that is slidable on the needle (12) and adapted to be immobilised in a position in which it covers the tip of the needle, characterized in that the protective sleeve (20) has a tubular projection (22) that surrounds the needle (12) and is configured to pierce the skin of the patient together with the needle (12).

## Description

The invention relates to a needle for piercing the skin of a patient, comprising a sleeve that is slideable on the needle and adapted to be immobilised in a position in which it covers the tip of the needle.

Medical syringes generally involve the risk that the medical personnel hurts itself at the needle and becomes infected. For this reason, it has been known to provide the needle with a protective sleeve that projects the tip of the needle after use.

DE 20 2009 011 253 U1 discloses a syringe with a needle of the type indicated above, that is specifically designated for use as a so-called permanent venous cannula. The cannula has a flexible catheter the proximal end of which is connected to an injection valve. For applying the cannula, the needle of the syringe is pushed through the catheter, and both are commonly introduced into the vein of the patient. Subsequently the syringe is withdrawn whereas the catheter and the injection valve remain at the body of the patient. Initially, the protective sleeve is in friction engagement with the injection valve, so that it will be displaced towards the region of the tip of the needle when the needle with withdrawn. A flexible tension member connects the protective sleeve with the casing of the syringe and thereby prevents the sleeve from being totally removed from the needle. Moreover, the protective sleeve includes a locking mechanism that prevents the sleeve from being pushed back on the needle into a position in which it exposes the tip of the needle.

In this known syringe, the protective sleeve may fulfil its function only when the syringe is used together with a permanent venous cannula.

WO 2006/010393 Al discloses a syringe wherein, after use, the needle can be withdrawn into the casing by withdrawing the piston of the syringe. Here, the tip of the needle is protected only on condition that the piston is withdrawn after the syringe has been used.

It is an object of the invention to provide a needle with improved operational safety.

In order to achieve this object, the protective sleeve of a needle of a type indicated above has a tubular projection that surrounds the needle and is adapted to pierce the skin of the patient together with the needle.

When this needle is used for injections, the skin is pierced with the needle while the latter is surrounded by the projection of the protective sleeve, so that the needle will penetrate through the skin of the patient together with the projection of the protective sleeve. When subsequently the needle is withdrawn again, the larger friction between the tubular projection and the tissue of the patient assures that the needle will be withdrawn alone and the protective sleeve is displaced towards the tip end of the needle. Only then will the sleeve be entrained with the needle, so that the projection is also withdrawn from the tissue. Then, the sleeve is immobilised on the tip of the needle in a position in which it reliably prevents that anybody is hurt by the tip of the needle. The projection of the protective sleeve may be made so flexible and/or so obtuse that it will not pierce the skin of the patient when it is no longer reinforced by the needle and the skin is not punctured by the needle tip.

Useful embodiments and further developments of the invention are indicated in the dependent claims.

For immobilising the protective sleeve on the tip of the needle a locking mechanism within the sleeve as well as a tension member that can only be extended to a limited length may be provided for connecting the sleeve with the casing of the syringe or with a connecting piece, the so-called Luer cone, with which the needle is coupled to the casing of the syringe, similarly as with the syringe that has been described above and serves for applying a permanent venous cannula. Optionally, the range of displacement of the protective sleeve towards the tip of the needle may also be delimited by a local enlargement of the needle.

In another embodiment, the protective sleeve may be connected to a thrust and tension member that is guided for sliding movement on the casing of the syringe and is adapted to be snap-fastened or locked at the casing in a position in which the protective sleeve covers the needle tip.

Embodiment examples will now be described in greater detail in conjunction with the drawings wherein:
- Fig. 1: shows the front end portion of a syringe with a needle according to an embodiment of the invention in an axial section;
- Fig. 2: shows the syringe of Fig. 1 in the condition after an injection;
- Fig. 3: shows an enlarged section of a protective sleeve for the needle of Figs. 1 and 2;
- Fig. 4: shows an enlarged section of a protective sleeve according to a modified embodiment;
- Figs. 5 and 6: are sectional views analogous to Figs. 1 and 2, but with the plane of the section rotated by 90° in comparison to Figs. 1 and 2;
- Fig. 7: is a cross-section along the line VII-VII in Fig. 5;
- Figs. 8 and 9: are sectional views analogous to Figs. 1 and 2 for a needle according to another embodiment;
- Fig. 10: is a cross-section along the line X-X in Fig. 7;
- Fig. 11: shows a protective sleeve with an arresting mechanism for a needle according to another embodiment;
- Fig. 12: is an enlarged top plan view of the arresting mechanism shown in Fig. 11;
- Fig. 13: shows a protective sleeve with an arresting mechanism for a needle according to yet another embodiment; and
- Fig. 14: is an enlarged top plan view of the arresting mechanism shown in Fig. 13.

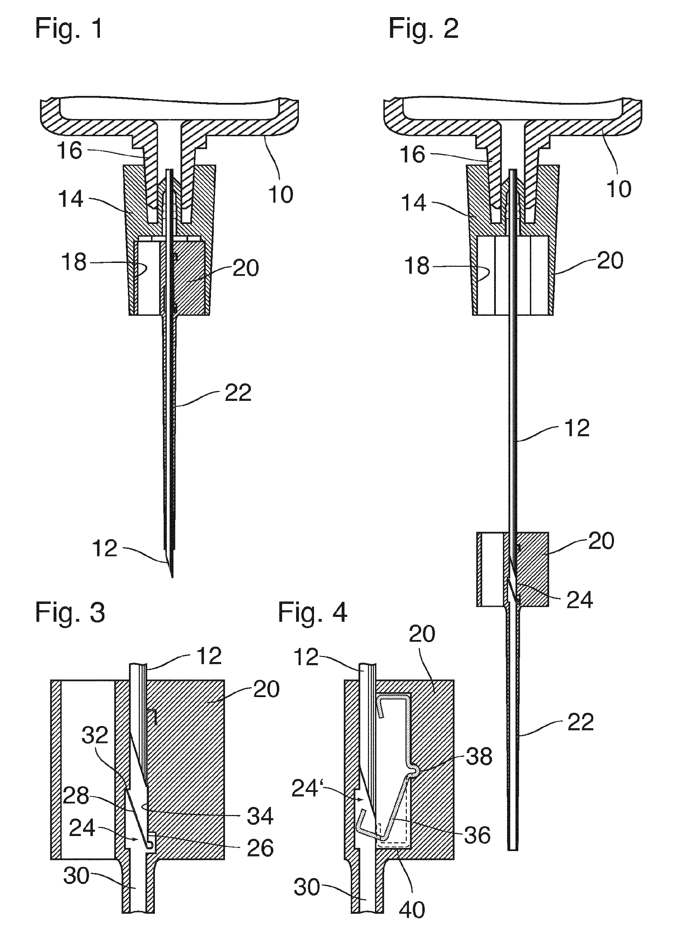

Fig. 1 shows the front end of a casing 10 of an injection syringe. A needle 12 of the syringe is held in a connecting piece 14 (Luer cone) that is coupled to a port 16 of the casing 10 in known manner.

The connecting piece 14 is formed at its front end with a recess 18 that accommodates a protective sleeve 20.

The protective sleeve 20 is slidabeably guided on the needle 12 and has a flexible tubular projection 22 that is made of plastics or metal and surrounds the needle 12 and extends almost over the entire length of the needle so that it ends only shortly behind the tip of the needle 12. The wall thickness of the projection 22 is reduced towards the free end so that the flexibility of this projection will increase towards the end unless it is reinforced by the needle 12 from the inside.

When an injection shall be administered to a patient, the skin is punctured with the tip of the needle 12, and the needle is inserted further, thereby causing also the projection 22 that is reinforced by the needle to penetrate into the tissue of the patient. When the injection has been completed, the syringe is withdrawn so that the needle is withdrawn from the skin of the patient. Since, however, the friction between the needle 12 and the projection 22 is smaller than the friction between the projection 22 and the tissue of the patient, the projection 22 gets stuck in the tissue so that the protective sleeve 20 will be displaced on the needle 12 until it has reached the position shown in Fig. 2 in which it surrounds and protects the tip of the needle.

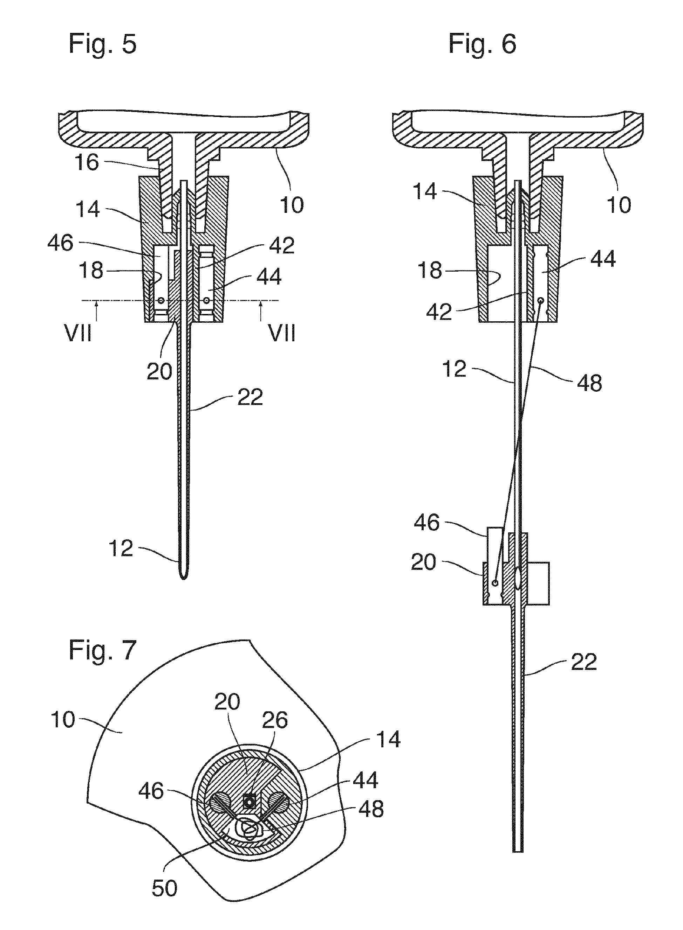

A mechanism that will be described later in greater detail in conjunction with Figs. 5 and 6 assures that the protective sleeve cannot be drawn off from the needle 12 in its entirety but remains in the position shown in Fig. 2. Therefore, when the needle 12 is withdrawn further, the projection 22 will also be drawn out of the skin of the patient.

The protective sleeve 20 includes a locking mechanism 24 that prevents the sleeve from being pushed back on the needle 12 in the direction towards the connecting piece 14. Thus, the protective sleeve 20 can reliably prevent that anybody is hurt or infected by the tip of the needle 12. When the needle 12 has been withdrawn, the tip of the projection 22 is weakened to such an extent that it does not constitute a risk of injury.

A possible construction of the locking mechanism 24 has been shown in Fig. 3. Here, the locking mechanism is formed by a spring 26 that is embedded in a massive part of the protective sleeve 20 and has a leg 28 that is obliquely turned back and traverses a guide passage 30 for the needle 12. The free end of the leg 28 gets caught at a step 32 formed in the wall of the guide passage 20 and thereby prevents the sleeve 20 from being thrust further onto the needle 12. In the initial condition of the syringe, shown in Fig. 1, the leg 28 is folded back onto the main part of the spring 26 and is thus accommodated in a pocket 34 such that is clears the guide passage 30. Only when the needle 12 has been drawn back relative to the sleeve 20 and the tip of the needle has passed the leg 28, this leg will spring back into the position shown in Fig. 3.

Fig. 4 shows a locking mechanism 24' according to an alternative embodiment. A spring 36 has two mirror-symmetric U-shaped legs that are connected to one another by an elastic joint 38. The spring 36 is accommodated in a chamber 40 of the protective sleeve 20 and is additionally secured against axial displacement in the protective sleeve by the joint 38. In the initial condition, in which the needle 12 completely fills the guide passage 30, the spring 36 assumes the position that has been shown in dashed lines in Fig. 4 and in which it clears the guide passage 30. When the needle is drawn back, the joint 38 causes the lower leg of the spring to tilt into the position shown in Fig. 4 in which the tip of the needle 12 gets caught if one tries to push back the protective sleeve 20. A portion of the top leg of the spring 36 that engages the needle 12 is bent at an angle of somewhat more than 90° so that it will not compromise the retreat movement of the needle 12 (upwards in Fig. 4).

Figs. 5 and 6 show the syringe in an axial section in which the sectional plane extends at right angles to the sectional plane in Figs. 1 and 2. This is why the locking mechanism 24 is not visible here. Instead, it can be seen that the connecting piece 14 forms a lug 42 that projects inwardly from the peripheral wall of the recess 18. A holding pin 44 has been inserted from the lower end of the connecting piece 14 to a bore of the lug 42 and has firmly been crimped in this bore. Similarly, another holding pin 46 is crimped in a massive part of the protective sleeve 20 that is arranged diametrically opposite to the lug 42. The two holding pins 44 and 46 are connected to one another by a filament 48 that is visible only in Figs. 6 and 7. As shown in Fig. 7, this filament 48 is nested in a chamber 50 formed inside of the protective sleeve 20. This chamber 50 connected to the bores for the holding pins 44 and 46 by slots that pass through in axial direction.

When the needle 12 is withdrawn after the injection, the protective sleeve 20 exits from the recess 18 of the connecting piece 14. This implies that the holding pin 46 is also drawn out of the connecting piece. The other holding pin 44, however, remains fixed in the connecting piece so that the filament 48 is extended. The length of the filament 48 has been selected such that the protective sleeve 20 can only be drawn out up to the position shown in Fig. 6 but cannot be drawn off from the needle 12 completely.

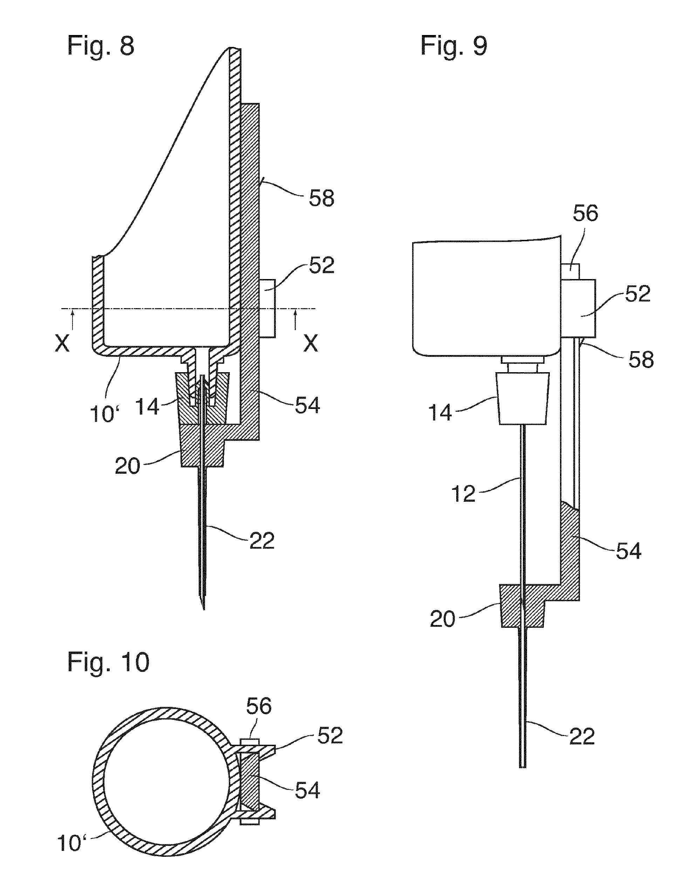

Figs. 8 to 10 illustrate a syringe according to another embodiment.

This syringe has a casing 10' on which, on the outer periphery, an axial guide 52 is formed for a thrust and tension member 54 that is connected to the protective sleeve 20. As is shown in Fig. 10, the thrust and tension member 54 has a dovetail profile and the guide 52 is formed by elastic ribs that have a claw-shaped profiles and between which the thrust and tension member 54 can be snapped-in laterally before the connecting piece 14 with the sleeve 20 carrying the needle 12 is coupled to the casing 10. At the top end, the thrust and tension member 54 forms an enlarged stop 56 that abuts at the top end of the guide 52 so as to limit the length to which the thrust and tension member 54 may be drawn out, as shown in Fig. 9. Moreover, the thrust and tension member 54 has a catch spring 58 that is initially located above the guide 52 as shown in Fig. 8 and can retreat elastically when it passes the guide 52. When the protective sleeve 20 reaches the position shown in Fig. 9, the spring 58 snaps back and is then caught at the lower end of the guide 52 so that it prevents the sleeve 20 from being pushed back.

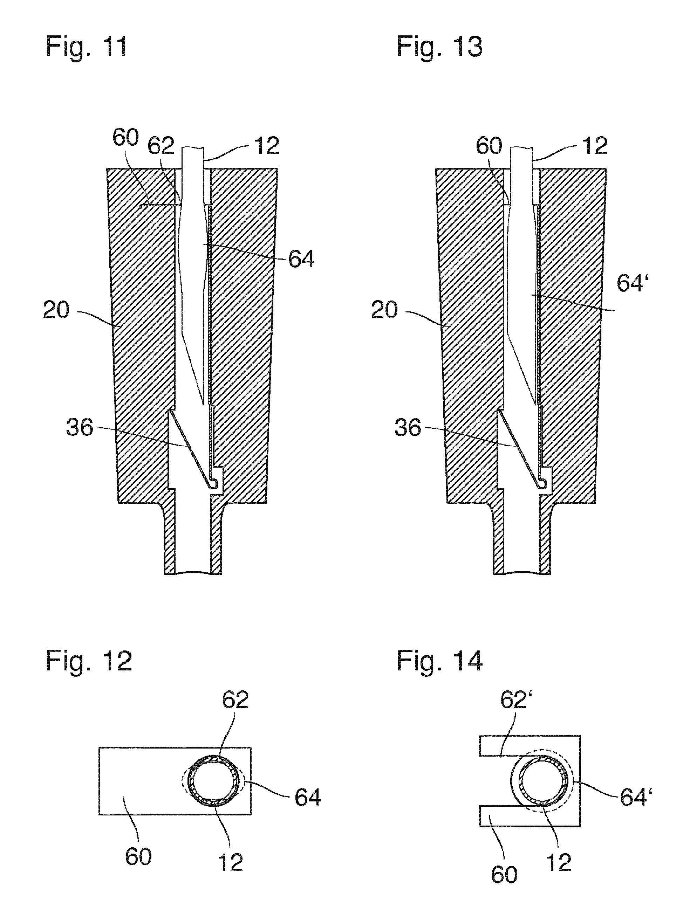

Figs. 11 to 14 show embodiment examples in which the protective sleeve has an arresting mechanism with which it can be arrested directly at the tip of the needle against movement in both directions.

The protective sleeve 20 in Figs. 11 and 12 has a similar construction as in Fig. 3. Only, the spring 26 is in this case provided with a tab 60 at its top end, and the tab traverses the guide passage 30 and has a circular hole 62 through which the needle 12 passes through. At a point shortly behind its tip, the needle has been compressed in a lateral direction so as assume a flattened shape, causing the needle to form an elliptic enlargement 64 in the transverse direction, which enlargement does not fit through the hole 62 and thus limits the range of displacement of the protective sleeve. The compression of the needle into the flat shape may be performed for example after the sleeve has been thrust onto the needle and into engagement with the connecting piece 14, without damaging the projection 22 that surrounds the flattened part of the needle.

In Figs. 13 and 14, the needle has a cylindrical enlargement 64' that extends to the tip of the needle. This enlargement may for example be formed by inserting a punch from the tip end into the needle. The tab 60 may in this case straddle the needle in a fork configuration and may have a U-shaped hole 62' that is open to the end of the tab.

Optionally, the enlargement 64 or 64' may also be locked at a constriction of the guide passage 30, which constriction is formed by the plastic material of the protective sleeve 20.

## Claims

1. A needle for piercing the skin of a patient, comprising a protective sleeve (20) that is slidable on the needle (12) and adapted to be immobilised in a position in which it covers the tip of the needle, **characterized in that** the protective sleeve (20) has a tubular projection (22) that surrounds the needle (12) and is configured to pierce the skin of the patient together with the needle (12).

2. The needle according to claim 1, wherein the range of displacement of the protective sleeve (20) in the direction of the tip of the needle (12) is limited by a flexible tension member (48), and the protective sleeve (20) has an arresting device (24; 24') which, in a position in which the sleeve (20) covers the tip of the needle, engages the needle tip and prevents the sleeve (20) from being pushed back.

3. The needle according to claim 2, wherein the flexible tension member (48) connects the sleeve (20) to a connecting piece (14) that is fixedly arranged at the distal end of the needle (12) and adapted to couple the needle to a casing (10) of a syringe.

4. The needle according to any of the preceding claims, having a connecting piece (14) that is fixedly arranged at the distal end of the needle (12) and adapted to couple the needle to a casing (10) of a syringe, said connecting piece having a recess (18) that accommodates the protective sleeve (20) in a position in which the free end of the projection (22) is flush with the tip of the needle (12) or retreats relative to this tip.

5. The needle according to claim 4, wherein the tension member (48) interconnects two holding pins (44, 46), one of which is firmly held in a bore of the sleeve (20) and the other of which is firmly held in a bore of the connecting piece (14).

6. The needle according to claim 1, wherein the range of displacement of the protective sleeve (20) in the direction towards the tip of the needle (12) is limited by a local enlargement (64; 64') of the needle (12), and the sleeve (20) has an arresting device (24; 24') which, in a position in which the sleeve (20) covers the needle tip, engages the needle tip and prevents the sleeve (20) from being pushed back.

7. A syringe having a needle according to claim 1, wherein, for immobilising the protective sleeve (20) in the position in which it covers the needle tip, a thrust and tension member (54) is provided that is rigidly connected to the protective sleeve (20) and is guided for axial displacement at a casing (10') of the syringe.
